# EUROPEAN PATENT SPECIFICATION

(11) **EP 3 374 497 B1**
(45) Date of publication and mention of the grant of the patent: **04.08.2021**
(21) Application number: 16864994.5
(22) Date of filing: 10.11.2016
(51) Int. Cl.: A61K 35/15, A61K 38/44, C12N 5/0786, C12N 5/078, C12N 15/00, A61P 35/00

(54) **MODIFIED MACROPHAGES FOR USE IN THE TREATMENT OF CANCER**
MODIFIZIERTE MAKROPHAGEN ZUR VERWENDUNG IN DER KREBSBEHANDLUNG
MODIFIED MACROPHAGES POUR L'UTILISATION DANS LE TRAITEMENT DU CANCER

(30) Priority: 11.11.2015 US 201562253836 P
(43) Date of publication of application: 19.09.2018
(73) Proprietor: Zhu, Zhenglun, Newton, MA 02459 (US)
(72) Inventor: Zhu, Zhenglun, Newton, MA 02459 (US)
(74) Representative: Purdylucey Intellectual Property
(86) International application number: PCT/US2016/061310
(87) International publication number: WO 2017/083508

(56) References cited:
- US-A1- 2003 035 790
- US-A1- 2008 248 011
- US-A1- 2012 183 553
- US-A1- 2015 297 679
- X. WU ET AL: "Homeobox Transcription Factor VentX Regulates Differentiation and Maturation of Human Dendritic Cells", JOURNAL OF BIOLOGICAL CHEMISTRY, vol. 289, no. 21, 23 May 2014 (2014-05-23) , pages 14633-14643, XP055139813, ISSN: 0021-9258, DOI: 10.1074/jbc.M113.509158
- JOSETTE M. NORTHCOTT ET AL: "Fighting the force: Potential of homeobox genes for tumor microenvironment regulation", BBA - REVIEWS ON CANCER., vol. 1855, no. 2, 1 April 2015 (2015-04-01), pages 248-253, XP055560701, NL ISSN: 0304-419X, DOI: 10.1016/j.bbcan.2015.03.004
- XIAOMING WU ET AL: "The homeobox transcription factor VentX controls human macrophage terminal differentiation and proinflammatory activation", JOURNAL OF CLINICAL INVESTIGATION, vol. 121, no. 7, 1 July 2011 (2011-07-01), pages 2599-2613, XP055560677, GB ISSN: 0021-9738, DOI: 10.1172/JCI45556
- H. GAO ET AL: "VentX, a Novel Lymphoid-Enhancing Factor/T-Cell Factor-Associated Transcription Repressor, Is a Putative Tumor Suppressor", CANCER RESEARCH, vol. 70, no. 1, 22 December 2009 (2009-12-22), pages 202-211, XP055108799, ISSN: 0008-5472, DOI: 10.1158/0008-5472.CAN-09-2668

## Description

### BACKGROUND

The role of immunity in oncogenesis has been increasingly appreciated. Macrophages are executors of both innate and adaptive immunity and have been recognized as key components of tumors and their microenvironment. Extensive investigations suggested the role of tumor-associated macrophages (TAMs) in the growth, invasion and metastasis of nearly all tumors. Derived from circulating monocytes, TAMs display a broad spectrum of phenotypes, ranging from the M1-like phenotype in early stages of selected tumors to the M2-like phenotype in most advanced tumors. Consistent with its role in promoting tumorigenesis, the M2-like TAMs display the characteristic phenotype of elevated expression of IL-10, IL4, MMP, and VEGF, but decreased expression of pro-inflammatory cytokines and cytotoxic iNOs and ROIs, which are implicated in tumoricidal activities. Besides its intrinsic function in promoting tumorigenesis, TAMs also contribute to the suppression of anti-tumor immunity by alternating T-cell responses and balance in the tumor microenvironment. The functional plasticity of TAMs was well recognized. However, the effectiveness and potential application of targeting TAMs in tumor treatment has been restricted by the limited understanding of how TAMs plasticity is controlled by cell intrinsic factors.

Wu et al, ("Homeobox Transcription Factor VentX Regulates Differentiation and Maturation of Human Dendritic Cells", Journal of Biological Chemistry, vol. 289, no. 21, 23 May 2014) describes the generation of monocytes overexpressing VentX.

Xiaoming Wu et al, ("The homeobox transcription factor VentX controls human macrophage terminal differentiation and proinflammatory activation", Journal of Clinical Investigation, vol. 121, no. 7, 1 July 2011) describes that the cytokines M-CSF, GM-CSF and IL-3 induce an elevated level of VentX in monocytes, whereby the monocytes are differentiated into macrophages.

Josette M. Northcott et al., ("Fighting the force: Potential of homeobox genes for tumor microenvironment regulation", BBA-Reviews on Cancer, vol. 1855, no. 2, 1 April 2015") describes that topical application of methylcellulose pellets containing HOX gene expression plasmids successfully delay tumor progression in a murine model of skin cancer.

H. Gao, et al., ("VentX, a novel lymphoid-enhancing factor/T-cell factor-associated transcription repressor, is a putative tumor suppressor", Cancer Research, vol. 70, no. 1, 22 December 2009) describes that transfecting acute lymphocytic leukemia cells (Reh cells) with VentX results in the inhibit4ion of the cell proliferation, demonstrating that VentX is a putative tumor suppressor.

### SUMMARY

Described herein is a method of treating a cancer. The method comprising: providing a genetically modified macrophage or monocyte that contains a nucleic acid sequence encoding a Hom-1 polypeptide or a fragment thereof that contains the Hom-1 homeobox domain, wherein the nucleic acid sequence is operably linked to a heterologous promoter and the modified macrophage or monocyte expresses the Hom-1 polypeptide or the fragment thereof; and administering the modified macrophage or monocyte to a subject with a cancer. The modified macrophage or monocyte is generated by introducing an exogenous expression construct into a macrophage or monocyte derived from the subject or another subject. The modified macrophage exhibits an M1 phenotype. The method can further include, prior to the administering step, detecting a lower level of Hom-1 expression in a tumor-associated macrophage in the subject as compared to a control.

Described herein is a method of treating a cancer that includes contacting a macrophage or monocyte with one or more agents that induce expression of Hom-1, whereby the expression level of endogenous Hom-1 in the macrophage or monocyte is higher than before the contacting step; and administering the thus contacted macrophage or monocyte to a subject with a cancer.

Also described herein is a method of treating a cancer that comprises contacting a macrophage or monocyte with an agent that induces the expression of an M1 gene or an agent that inhibits the expression of an M2 gene, whereby a macrophage that exhibits an M1-phenotype is generated; and administering the thus generated macrophage to a subject with cancer.

Also described herein is a method of identifying a cancer in a subject. The method includes detecting the expression level of a gene in a macrophage from the microenvironment of a tissue area suspected of being a cancer, the gene being a Hom-1 gene, M1 gene, or M2 gene, wherein detecting (i) a lower level of the Hom-1 gene or M1 gene or (ii) a higher expression level of the M2 gene as compared to a corresponding control level indicates that the suspected tissue area is a cancer or is at risk of becoming a cancer.

A method of identifying a candidate compound for treating a cancer is also described in this disclosure. The method includes contacting a test cell with a test compound, wherein the test cell is a macrophage or monocyte; detecting in the test cell the expression level of (i) Hom-1, (ii) a reporter gene operably linked to a Hom-1 promoter, (iii) an M1 gene, or (iv) an M2 gene; and selecting a test compound that alters the expression level as compared to a corresponding control level, wherein the selected compound is a candidate compound for treating cancer. The method can further include contacting a protumor macrophage with the selected compound and assaying the thus contacted protumor macrophage for an anti-tumor activity. The protumor macrophage can be a tumor-associated macrophage or M2 macrophage. The contacting step can be performed with the test cell in a co-culture containing a cancer sample. The test cell can be selected from an M1 macrophage, M2 macrophage, tumor-associated macrophage, tissue macrophage, and monocyte-derived macrophage.

Also described herein is a method of identifying a candidate compound for treating a cancer that includes: providing a co-culture containing a test cell and a cancer sample, wherein the test cell is a macrophage or monocyte; adding a test compound to the co-culture; and selecting a test compound that, as compared to a control, (i) inhibits the cancer sample, (ii) increases the expression level of Hom-1 or a reporter gene operably linked to a Hom-1 promoter in the test cell, (iii) increases the expression of an M1 gene in the test cell, (iv) decreases the expression of an M2 gene in the test cell, or (v) inhibits a significant decrease of the expression level of Hom-1 or a reporter gene operably linked to a Hom-1 promoter in the test cell; wherein the selected compound is a candidate compound for treating cancer. The cancer sample is a cancer tissue sample. In the co-culture, the test cell and cancer sample can be in direct contact or in indirect contact with each other. The test cell can be selected from an M1 macrophage, M2 macrophage, tumor-associated macrophage, tissue macrophage, and monocyte-derived macrophage.

Further described herein is a method of identifying a candidate compound for treating a cancer that includes: providing a cancer tissue sample, contacting the tissue sample with a test compound, and selecting a test compound that, as compared to a control, (i) inhibits the tissue sample, (ii) increases the expression level of Hom-1 in a tumor-associated macrophage or monocyte in the tissue sample, (iii) increases the expression of an M1 gene in a tumor-associated macrophage or monocyte in the tissue sample, (iv) decreases the expression of an M2 gene in a tumor-associated macrophage or monocyte in the tissue sample, or (v) inhibits a significant decrease of the expression level of Hom-1 in a tumor-associated macrophage or monocyte in the tissue sample; wherein the selected compound is a candidate compound for treating cancer.

The invention relates a macrophage or monocyte for use in treating a subject with a cancer, wherein the macrophage of or monocyte is induced *ex vivo* to express an elevated level of endogenous Hom-1, or wherein the macrophage or monocyte is genetically modified to express an elevated level of Hom-1, to exhibit anti-tumor activity.

The details of one or more embodiments are set forth in the description below. Other features, objects, and advantages of the embodiments will be apparent from the description and from the claims.

### DETAILED DESCRIPTION

It was surprisingly discovered that Hom-1 expression in TAMs is significantly decreased in comparison with macrophages isolated from normal tissues. It was further discovered that, also surprisingly, increasing the expression of Hom-1 in TAMs converted them to M1-like macrophages with tumoricidal activities.

Hom-1, a human homeobox transcriptional factor, is an antagonist of the canonical Wnt signaling. A nucleic acid sequence of Hom-1 (SEQ ID NO:1) and the amino acid sequence (SEQ ID NO:2) it encodes are shown below:

Described herein is a method of treating a cancer in a subject by administering to the subject macrophages that exhibit anti-tumor activities. As used herein, unless otherwise specified, the terms "macrophages that exhibit anti-tumor activities," "M1-like macrophages," and "macrophages that exhibit an M1 phenotype" may be used interchangeably.

M1-like macrophages can be produced by (1) increasing Hom-1 expression in macrophages or monocytes, (2) increasing expression of one or more M1 genes in macrophages or monocytes, and/or (3) inhibiting expression of one or more M2 genes in macrophages or monocytes.

Monocytes (e.g., derived from a subject's peripheral blood) can be used in the method as it was shown that Hom-1 expression is both necessary and sufficient for monocyte-to-macrophage differentiation. In other words, increasing the expression of Hom-1 in monocytes can drive them to differentiate to macrophages.

Macrophages or monocytes can be induced *ex vivo* to express a higher level of endogenous Hom-1. Various agents or treatments can be used to induce Hom-1 expression, e.g., LPS, cholera toxin (CTX), chemotherapeutic agents, radiation, cytokines (e.g., GM-CSF), phorbol 12-myristate 13-acetate (PMA), and antibodies or RNAi against inhibitors of Hom-1 expression.

Macrophages or monocytes that have been genetically modified to express an elevated level of Hom-1 can also be used to treat a subject with cancer. For example, the genetically modified macrophages or monocytes can contain a nucleic acid sequence encoding a Hom-1 polypeptide or a fragment thereof that includes the Hom-1 homeobox domain. The nucleic acid sequence is operably linked to a promoter and the modified macrophages or monocytes express the Hom-1 polypeptide or the fragment thereof. Such modified macrophages or modified monocytes (which would differentiate to macrophages) express a sufficiently high level of Hom-1 to exhibit anti-tumor activities and/or an M1 phenotype.

Genetically modified macrophages or monocytes can also be generated by introducing extra copies of the Hom-1 gene into the macrophages or monocytes. For example, an expression construct containing a Hom-1 nucleic acid sequence (encoding a Hom-1 polypeptide or a fragment thereof that includes the Hom-1 homeobox domain) operably linked to the endogenous Hom-1 promoter can be introduced into macrophages or monocytes.

A Hom-1 polypeptide or fragment thereof that includes the Hom-1 homeobox domain can also be introduced into macrophages or monocytes by direct peptide delivery.

Methods known in the art can be used to genetically modify macrophages and monocytes. For example, an exogenous expression construct for expressing Hom-1 can be introduced (e.g., stably or transiently transfected into) macrophages or monocytes. In one embodiment, the Hom-1 nucleic acid sequence is operably linked to a heterologous (i.e., not a Hom-1 promoter) constitutive or inducible promoter. In one embodiment, the Hom-1 nucleic acid sequence is operably linked to an endogenous promoter.

M1-like tumoricidal macrophages can also be generated by inducing expression of M1 genes in macrophages or monocytes. Agents that can induce M1 genes include, but not limited to, LPS, CTX, PMA, GM-SCF, INFγ, and chemotherapeutic agents. Macrophages or monocytes can also be genetically modified to express elevated levels of M1 genes. M1 genes include IL1b, IL6, IL12, IL23, TNFα, iNOs, CD40, CD80, CD86, CD68, TLR4, TLR2, IL-1R, MHCII, CCL15, CCL20, CXCL9, CXCL1, and SOCS3.

Inhibiting expression of M2 genes in macrophages or monocytes can also produce M1-like tumoricidal macrophages. Agents that inhibit M2 genes include anti-IL4 agents (e.g., antibodies or RNAi agents), anti-IL13 agents (e.g., antibodies or RNAi agents), antibodies against M2 proteins, and RNAi agents targeting M2 genes. M2 genes include ARG1, MMP9, CCL18, VEGF, IL10, IL4, TGFb, CD163, CD206, CD68.,TLR8, TLR1, MHCII, TGM2, DcoyR, IL-1RII, Ym1/2, MMR/CD206, and SR.

Heterologous or autologous macrophages or monocytes can be used to generate M1-like macrophages. If heterologous macrophages or monocytes are used, HLA-matching can be conducted to avoid or minimize host reactions. HLA un-matched macrophages or monocytes may also be used. Autologous macrophages or monocytes can be obtained from a cancer patient using methods known in the art.

The generated M1-like macrophages can be administered to a subject through infusion or injection (for example, via intravenous, intrathecal, intramuscular, intraluminal, intratracheal, intraperitoneal, intracranial, subcutaneous, or another type of intra tissue route), transdermal administration, or other routes known in the art. In one example, the macrophages or monocytes can be directly injected at a site or into a tissue (e.g., liver or pancreas) or its surrounding area, where a tumor is found.

The subject can be treated with the M1-like macrophages as often (e.g., every 1 to 30 days) and as many times (e.g., 1-30 times) as needed to treat the cancer. The M1-like macrophages described herein can also be used in a combination therapy with other cancer treatments such as radiation, chemotherapy, antibodies, and small molecules drugs.

The data described below show that Hom-1 expression converts TAMs into tumoricidal cells independent of tumor types. Hence, any cancer can be treated using the M1-like macrophages described herein, particularly cancers associated with TAMs that express a low level of Hom-1. Before treating a subject with M1-like macrophages as described above, it may be useful to determine whether the Hom-1 expression level in TAMs in the subject is lower than that found in a control (e.g., a macrophage found in a normal tissue in the subject).

Examples of cancer that can be treated with M1-like macrophages include, but are not limited to, carcinoma and sarcoma such as leukemia, sarcoma, osteosarcoma, lymphomas, melanoma, glioma, glioblastoma, pheochromocytoma, hepatoma, ovarian cancer, skin cancer, testicular cancer, gastric cancer, pancreatic cancer, renal cancer, breast cancer, prostate cancer, colorectal cancer, cancer of head and neck, brain cancer, esophageal cancer, bladder cancer, adrenal cortical cancer, lung cancer, bronchus cancer, thyroid cancer, endometrial cancer, nasopharyngeal cancer, cervical cancer, liver cancer, metastatic cancer, and cancer of unknown primary site.

Detecting a lower expression level of Hom-1 or an M1 gene or a higher expression level of an M2 gene in macrophages found in the microenvironment of a tissue area as compared to that of a control (e.g., a corresponding level in a macrophage in a normal tissue) indicates that the tissue area is a cancer or is at risk of becoming a cancer. Thus, also contemplated herein is a method for identifying whether a suspected tissue area is a cancer or at risk of becoming a cancer.

Further described herein are methods of identifying a candidate compound for treating cancer. A test cell (i.e., a macrophage or monocyte) can be contacted with a test compound and the expression level of (i) Hom-1, (ii) a reporter gene operably linked to Hom-1 promoter, (iii) an M1 gene, (iv) a reporter gene operably linked to an M1 promoter, (v) an M2 gene, or (vi) a reporter gene operably linked to an M2 promoter in the test cell is detected. A test compound that increases the expression level of any of (i)-(iv), and/or decreases the expression level of (v) or (vi) as compared to a control (e.g., a corresponding level in a test cell not contacted with the test compound) is a candidate compound for treating cancer.

In one screening method, a test compound is added to a co-culture containing a test cell and a cancer sample. A test compound is a candidate compound for treating cancer if it, as compared to a control, (i) increases the expression level of Hom-1, a reporter gene operably linked to a Hom-1 promoter, an M1 gene, or a reporter gene operably linked to an M1 promoter in the test cell, (ii) inhibits a significant decrease of the expression level of Hom-1, a reporter gene operably linked to a Hom-1 promoter, an M1 gene, or a reporter gene operably linked to an M1 promoter in the test cell, or (iii) decreases the expression level of an M2 gene or a reporter gene operably linked to an M2 promoter in the test cell.

In a co-culture system, whether a test compound has an inhibitory effect on the cancer sample can also be determined. A test compound that inhibits the cancer sample (e.g., inhibits growth of a cancer cell, kills a cancer cell, or decreases the size of the cancer sample) as compared to a control is a candidate compound for treating cancer. The test cell and the cancer sample can be in direct contact with each other. Alternatively, the test cell and the cancer sample are not in direct contact (e.g., with the use of transwell inserts). The cancer sample can be a sample containing a cancer cell, for example, a cancer tissue sample, a cancer cell isolated from a cancer tissue sample, or a cell of a cancer cell line. Cancer tissue samples can be obtained from surgically dissected specimens from cancer patients. Such cancer tissues samples may contain TAMs.

A screening method can also be performed with a cancer tissue sample in the absence of a test cell. A cancer tissue sample can be contacted with a test compound. After a time period, TAMs can be isolated from the cancer tissue sample and the expression level of Hom-1, an M1 gene, or an M2 gene in the TAMs can be determined. Alternatively or in addition to, the expression level of Hom-1 in the tissue sample can be determined. A test compound is a candidate compound for treating cancer if it, as compared to a control, (i) increases the expression level of Hom-1 or an M1 gene, (ii) inhibits a significant decrease of the expression level of Hom-1 or an M1 gene, and/or (iii) decreases the expression level of an M2 gene. A test compound that inhibits the cancer tissue sample (e.g., decreases the size of the sample) as compared to a control is also considered as a candidate compound for treating cancer.

Test compounds to be screened (e.g., proteins, peptides, peptidomimetics, peptoids, antibodies, RNAi, small molecules, or other drugs) can be obtained using a method known in the art.

In any of the methods described herein, the expression level of Hom-1, an M1 gene, or an M2 gene can be determined at either the mRNA level or at the protein level. Promoter activities can also be measured. Methods of measuring mRNA levels, protein levels, and promoter activities are well known in the art.

In any of the above-described screening methods, the test cell can be a macrophage or monocyte. The macrophage can be an M1 macrophage, an M2 macrophage, a tumor-associated macrophage, a tissue macrophage, or a monocyte-derived macrophage. The test cell can also be a monocyte.

The specific example below is to be construed as merely illustrative, and not limitative of the remainder of the disclosure in any way whatsoever. Without further elaboration, it is believed that one skilled in the art can, based on the description herein, utilize the present disclosure to its fullest extent.

### EXAMPLE

We identify a role of the human homeobox protein Hom-1 in functional polarization of human macrophages. We found that Hom-1 promotes and is required for M1 activation of human macrophages, but not required for the expression of critical genes involved in M2 activation.

Using primary TAMs isolated from cancers, we found that Hom-1 expression in TAMs was significantly decreased in comparison with macrophages isolated from normal control tissues. We showed that the expression profile of Hom-1 in TAMs correlated with TAM phenotypes. Moreover, ectopic expression of Hom-1 converted TAMs into M1-like phenotype. Both *in vitro* and *in vivo* data showed that Hom-1 conferred tumoricidal activity to TAMs. Taken together, our studies demonstrated that Hom-1 converted TAMs into tumoricidal cells. We showed that Hom-1-expressing TAMs (exhibiting an M1 phenotype) exerted strong inhibitory effects on the growth of a variety of cancers, suggesting the role of Hom-1-modulated TAMs as a new modality in the treatment of cancers.

### Hom-1 expression was decreased in TAMs

In advanced tumors, TAMs display a protumor M2-like phenotype. See, Bronte and Murray (2015), Nat Med 21, 117-119. The plasticity of TAMs has been well appreciated, and a variety of cytokines have been implicated in the polarization of TAMs towards M2 phenotype. See, Noy and Pollard (2014), Immunity 41, 49-61. In comparison, the transcriptional machinery that controls the TAM polarization remains largely unknown.

Discarded surgical specimens from colon cancer resections were used to isolate TAMs from tumor tissues as well as macrophages from normal mucosa 15 cm away from the tumor sites as described below. As previously noted, FACS analysis showed that, in comparison to macrophages isolated from normal control mucosa, TAMs expressed significant higher levels of cell surface markers associated with M2 phenotypes, such as the CD68, CD163, CD206. See, Zhang et al. (2013), Eur J Cancer 49, 3320-3334. We found that there was no significant difference in the expression of non-discriminating macrophage marker CD33 in TAMs and control macrophages.

To determine whether Hom-1 may play a role in TAMs, we quantified Hom-1 expression in TAMs by qRT-PCR and found that, in comparison with its expression in control macrophages, Hom-1 expression is significantly decreased in TAMs.

### VentX regulated TAM plasticity and polarized TAMs toward an M1 phenotype

Previous studies indicated that TAMs can be induced by LPS to display an M1 phenotype. See, Zhang et al. To determine whether Hom-1 plays a role in TAM plasticity, we examined Hom-1 expression in TAMs exposed to LPS. We found that Hom-1 expression was significantly elevated in TAMs after being stimulated with LPS. Parallel with the elevated expression of Hom-1 and consistent with prior findings, LPS stimulation of TAMs led to elevated secretion of inflammatory cytokines and cytotoxic iNOs.

To determine whether Hom-1 plays a regulatory role of TAM plasticity, we examined the effects of Hom-1 knockdown on TAM phenotype. Treatment of TAMs with anti-Hom-1 morpholinos (MO) led to around an 80% reduction of Hom-1 expression. Consistent with Hom-1's role as a key regulatory of TAM plasticity, we found that Hom-1 MO aborted LPS induced secretion of inflammatory cytokines and cytotoxic iNOs in TAMs. CD206 is a mannose receptor and a M2 cell surface marker that is highly expressed in TAMs. Reflecting the plasticity of TAMs, exposure of TAMs to LPS led to a significant reduction of CD68+CD206+ population and an increase of the CD68+CD206- cell numbers. Hom-1 MO abolished both effects of LPS on TAMs (p < 0.01).

The correlation between Hom-1 expression levels and TAM phenotypes prompted us to further explore the idea that Hom-1 controls TAM plasticity. TAMs were isolated and transfected with a plasmid encoding GPF-Hom-1 or control GFP. Compared with the control GFP transfected TAMs, TAMs transfected with GFP-Hom-1 displayed characteristics M1 morphology with elongated/fibroblast-like cell shape. FACS analysis showed that surface expressions of M1 markers CD40, CD80, and CD86 were significantly increased in TAMs transfected with GFP-Hom-1. In addition, secretions of pro-inflammatory cytokines TNFα, IL-1β, and IL-12 were significantly increased, while secretion of M2 cytokine IL-10 was significantly decreased in TAMs transfected with GFP-Hom-1. Consistently, gene expression analysis showed that M1 genes, such as IL-1β, IL-6, TNF-α, and iNOs increased significantly in TAMs transfected with GFP-Hom-1, while M2 genes, such as, CCL18, MMP9, VEGFA, and Arg1 decreased significantly in TAMs transfected with GFP-Hom-1. Taken together, our data suggested that Hom-1 regulated TAM plasticity and promoted M1 polarization of TAMs.

### Hom-1 converted TAMs into tumor suppressing cells

Our findings that Hom-1 promotes M1 polarization of TAMs prompted us to explore whether Hom-1-modified TAMs could exert tumor suppression. Freshly isolated TAMs from colon cancers were transfected with plasmid encoding GPF-Hom-1 or control GFP. The modified TAMs were then co-cultured with tumor or normal tissues of the same patient, using the trans-well culture system. Remarkably, after 7-10 days of co-culturing, tumor volumes were significantly decreased (around 70%) during the incubating with GFP-Hom-1 modified TAMs (p <0.01), while there was no significant change of size in tumors incubated with GFP-transfected TAMs or with TAMs alone. To determine whether the shrinkage of tumor volume was related to reduction of cancer cells, we performed tissue section, H&E staining and immunohistochemistry of colon cancer cells with CK20 antibody. We found that CK20 positive tumor cells appeared in nests, cords, and sheets in the tumors incubated with TAMs or GFP modified TAM. However, CK20 positive tumor cells disappeared during the incubation with TAMs transfected with GFP-Hom-1. The specificity of tumoricidal effects of Hom-1-modified TAMs was demonstrated by the findings that Hom-1-modified TAMs exerted minimal effects on the volume and morphology of normal colon mucosa during the incubation with TAMs transfected with either GFP or GFP-Hom-1.

### Hom-1 promotes tumoricidal function of TAMs in vivo

Our findings that Hom-1 converted TAMs into tumoricidal cells *in vitro* prompted us to explore the potential role of Hom-1-regulated TAMs in tumorigenesis *in vivo.* Colon cancers were cut into around 0.5 cm pieces and surgically seeded in the subcutaneous space of the abdominal side of NSG mice. One week later, MO-Hom-1 transfected TAMs (Hom-1 inhibited) or GFP-Hom-1 transfected TAMs (Hom-1 expressing) were injected through mouse tail vain. After 8 weeks of xenograft, tumors developed in mice injected with MO-Hom-1 transfected TAMs, but not in mice injected with GFP-Hom-1 transfected TAMs.

We also found that the TAMs or monocytes can be induced to exhibit an M1 phenotype by culturing them in an M1 differentiation media. These M1-differentiated TAMs/monocytes can be infused into NSG mice and inhibit cancer growth *in vivo.* The effects of M1 differentiated TAMs on tumor growth are abolished by inhibition of Hom-1 expression in these TAMs or monocytes.

### Effects of ectopic expression of Hom-1 in TAMs on various cancer types

TAMs have been implicated in oncogenesis of essentially all tumors. Following our studies on TAMs in colon cancer cells, we extended our investigation to other tumor types.

Surgical species of lung, melanoma, esophagus, gastric, and pancreatic cancers were obtained, and TAMs were isolated as described above. Macrophages from corresponding normal tissues of the same patient were obtained. Hom-1 expression in TAMs and tissue macrophages were quantified using real time RT-PCR. Hom-1 expressions in TAMs of all these tumors were low in comparison to Hom-1 expression in corresponding macrophages from distant normal tissues.

To determine whether Hom-1 could convert these TAMs into tumoricidal cells, GFP or GFP-Hom-1 were transfected into the TAMs. After 48 hours of transfection, GFP positive cells were sorted out and co-cultured with individual tumors. Tumor volumes of all tumors decreased during co-culture with GFP-Hom-1 transfected TAMs but not with the control GFP-transfected TAMs. Our results suggested that Hom-1 could convert TAMs into tumoricidal cells independent of tumor types.

### Collection of colon tissue samples

Cancer tissues and normal tissues were obtained from surgically dissected specimens from patients in pathology lab. Around 5-10 gram of tissue was collected from each tumor mass, or from normal mucosa at 15 cm away from tumor mass. Patient blood samples were also collected.

### Preparation of intraepithelial lymphocyte

Lamina propria mononuclear cells (LPMCs) were isolated using previously described techniques with modification (Kamada N, et al, 2008; Pignata C, et al, 1990). In brief, dissected fresh mucosa and tumor mass were rinsed in 10-cm Petri dish with Ca²⁺-free and Mg²⁺-free hank's balanced salt solution (HBSS) (life technologies) containing 2% fetal bovine serum (FBS) and 1 mM Dithiothreitol (DTT) (Sigma-Aldrich) to remove mucus. The mucosa and tumor were cut into 0.5 cm pieces by a razor blade and incubated in 6-well plate with 5 mL HBSS containing 1 mM EDTA (Sigma-Aldrich) at 37°C for 1 hour, then passed through a gray-mesh (100 micron). The flowthrough contains intraepithelial lymphocyte and epithelial cells and was analysis by a flow cytometer.

### Isolation of macrophage from tumor mass and normal mucosa

Subsequently, the mucosa and tumor were incubated in HBSS (with Ca²⁺ and Mg²⁺), containing 2% FBS, 1.5 mg/mL Collagenase D (Roche), 0.1 mg/mL Dnase I at 37°C for 1 hour. Digested tissues were passed through a gray-mesh (70 micron) filter. The flowthrough was collected and resuspended in a 40% Percoll solution (Pharmacia), then layered on 60% Percoll, and centrifuged at 2000 rpm for 30 min without brake. LPMCs at the interface were collected. Normal mucosal macrophages and TAMs were purified from LPMCs using EasySep™ Human Monocyte/Macrophage Enrichment kit without CD16 depletion (StemCell Technologies), according to the manufacturer's instructions. Cells isolated by these techniques were routinely more than 98% viable by propidium iodide (PI) staining. The purity of intestinal macrophages was more than 95%.

### Preparation of macrophages from peripheral blood

Peripheral blood mononuclear cells (PBMC) from healthy adult donors at Brigham and Women hospital were isolated by Ficoll density gradient centrifugation. Human monocytes were purified from PBMCs using EasySep™ Human Monocyte Enrichment kit without CD16 depletion according to the manufacturer's instructions. Purified cells were cultured in completed RPMI medium with 10 ng/mL of M-CSF (PeproTech). After enrichment, monocytes were cultured in completed RPMI medium with M-CSF for 5 days, cells were used for co-culture system.

### FACS analysis

Phenotypic analysis of TAMs and other lymphocytes was performed using flow cytometry after immunolabeling of cells with fluorescence dye-conjugated antibodies. The following antibodies were used: PE-conjugated anti-CD3 (OKT3), -CD25 (BC96), -CD14 (61D3), -CD68 (eBio Y182A), -CD163 (eBio GH161), -CD206, FITC-conjugated anti-CD4 (RPA-T4), -CD33 (HIM3-4), APC-conjugated anti-CD8 (OKT8), -CD4 (OKT4) (eBioscience, Inc). Intracellular staining of Foxp3 (236A/E7), IFN-γ, Perforin, and Granzyme B was performed with PE-conjugated antibodies following the protocol provided by manufacturer. Isotope control labeling was performed in parallel. Antibodies were diluted as recommended by the supplier. Labeled cells were collected on FACScan flow cytometer with Cell-Quest software (BD Biosciences) and analyzed by FlowJo software. Results are expressed as the percentage of positive cells.

### Organotypic co-cultures of tumor and macrophages

Transwell inserts (0.4 µm pore sized, Costar, Corning) were placed in 12-well polystyrene tissue culture plates (Becton Dickinson, Franklin Lakes, NJ). Mucosa and tumor mass were weighed and washed with 1x PBS buffer, plus antibiotics, then cut into 0.5 cm pieces. Around 50 mg of tissues were seeded in the upper compartment of a 12-well transwell and filled with 0.5 mL of RPMI 1640 completed medium. 5 x 10⁵ of TAMs were added to the lower compartment at the density of 0.5 million cells/well with no direct cell-tissue contact and filled with 2 mL of PRMI completed medium. The plate incubated at 37°C, 5% CO₂. 0.5 mL of culture medium were collected for cytokine analysis and fresh medium were added every three days. After two weeks co-culture, low chamber macrophages were collected and total RNA was isolated by the TRIzol reagent (Ambion). Tumor and normal mucosal were monitored two times a week by calipers to calculate tumor volumes according to the formula (length x width²) / 2. The photos of tissues were taken by a 3-megapixel CMOS camera on Leica EZ4D stereomicroscope or a digital camera on iPhone.

### Immunohistochemistry

Tumors or normal tissues were fixated in Formalin (Fisher Scientific Company, Kalamazoo, MI). CK20 stainings (Dako, Carpinteria, CA, clone Ks20.8, 1:50) and Haematoxylin/eosin (H&E) stainings were performed. CK20 staining was performed on the Leica Bone III staining platform using Epitope Retrieval 2 for 20 minutes online, and using Bone Polymer Refine detection kit. Microscopic analysis was performed with a Nikon Eclipse Ti fluorescence microscopy. Images were captured at an original magnification of 40 x using a color camera applying the NIS Elements imaging software (Nikon). Brightness and contrast for representative images were adjusted equally among groups.

### Hom-1 overexpression

Transfection of GFP-Hom-1 into blood macrophages and TAMs was carried out through lipofectamine 2000 (Life technologies) according to manufacture protocol. 48 hours after transfection, cells were filtered through a 70 um filter for cell sorting. GFP positive cells were sorted by BD FACSAria II under the Baker Bio-Protect Hood in a sterile condition. After sorting, cells were cultured in RPMI 1640 completely medium.

### Hom-1 knockdown

Colon TAMs or human primary monocytes were transfected with Morpholino (MO) antisense oligonucleotides using the Human Monocyte Nucleofector Kit (Lonza, Walkersville, MD) according to the manufacturer's instructions. Briefly, 5×10⁶ cells were re-suspended into 100 µl nucleofector solution with 2.5 nmol of either Hom-1 MO oligonucleotides or a standard control MO oligonucleotides and electroporated with the Nucleofector II Device (Lonza). Cells were then immediately removed from the device and incubated overnight with 1ml pre-warmed Human Monocyte Nucleofector Medium containing 2mM glutamine and 10% FBS. Cells were then re-suspended into complete RPMI medium and treated with appropriate cytokines to induce differentiation into macrophages. All the MO oligonucleotides were ordered from Gene Tools (Philomath, OR).

### Cytokine measurement

Levels of IL-1β, IL-10, TNF-α and IL-12p70 in the supernatants of *E. coli* LPS-(Sigma-Aldrich) treated blood macrophages or LPS-treated TAMs were quantified using ELISA kits obtained from eBiosciences. Analyses were conducted according to the manufacturer's instructions.

### qRT-PCR

Total RNA was isolated by the TRIzol reagent and RNA amounts were measured by NanoDrop 2000 (Thermo Scientific). An equal amount of RNA was used for first-strand cDNA synthesis with SuperScript III First-Strand Synthesis System (Life Technologies) according to the manufacturer's protocol. To amplify Hom-1 cDNA with conventional PCR, we used the AccuPrime Taq DNA polymerase system (Life Technologies) following the manufacturer's instructions. PCR products were separated on 2% agarose gels and stained with ethidium bromide. GAPDH was used as an internal control. We performed quantitative measurement of Hom-1 and other genes cDNA with SYBR Green on a LightCycler (480 Real-Time PCR System; Roche). Relative expression profiles of mRNAs were then calculated using the comparative Ct method (DDCT method).

### Arginase activity and NO assays

Arginase activity was quantified in cell lysates by measuring the production of urea using the QuantiChrom arginase Assay Kit (DARG-200; BioAssays Systems). Nitrite concentrations in culture supernatants were determined using Griess reagent kit (Molecular Probes).

### Statistical Analysis

Student's test was used for statistical analysis.

## Claims

1. A macrophage or monocyte for use in treating a subject with a cancer, wherein the macrophage or monocyte is induced *ex vivo* to express an elevated level of endogenous Hom-1, or wherein the macrophage or monocyte is genetically modified to express an elevated level of Hom-1, to exhibit anti-tumor activity.

2. The macrophage or monocyte for use of Claim 1, wherein the macrophage or monocyte is generated by introducing an exogenous expression construct into a macrophage or monocyte derived from the subject with the cancer, or by introducing an exogenous expression construct into a heterologous macrophage or monocyte that is optionally HLA matched.

3. The macrophage or monocyte for use of Claim 1 or Claim 2, wherein the macrophage or monocyte is genetically modified by an exogenous nucleic acid sequence encoding a Hom-1 polypeptide or a fragment thereof that contains the Hom-1 homeobox domain, wherein the nucleic acid sequence is operably linked to either an endogenous Hom-1 promoter, or to a heterologous promoter, wherein the heterologous promoter is optionally a constitutive promoter or an inducible promoter.

4. The macrophage or monocyte for use of Claims 1 to 3, wherein the macrophage or monocyte is induced *ex vivo,* optionally by LPS, cholera toxin (CTX), a chemotherapeutic agent, radiation, a cytokine (e.g., GM- CSF), phorbol 12-myristate 13-acetate (PMA), an antibody or an RNAi against an inhibitor of Hom-1 expression, to express a higher level of endogenous Hom-1.

5. The macrophage or monocyte for use of Claims 1 to 4, wherein the macrophage or monocyte exhibit an M1 phenotype or expresses elevated levels of M1 genes such as IL1β, IL6, IL12, IL23, TNFα, iNOs, CD40, CD80, CD86, CD68, TLR4, TLR2, IL-1R, MHCII, CCL15, CCL20, CXCL9, CXCL1, or SOCS3.

6. The macrophage or monocyte for use of Claims 1 to 5, wherein the subject with the cancer, prior to said treating, is determined to express a lower level of Hom-1 expression in a tumor-associated macrophage (TAM) when compared to a control.

7. The macrophage or monocyte for use of Claims 1 to 6, which is a protumor macrophage, such as a tumor-associated macrophage (TAM) or a M2 macrophage, prior to induced or genetically modified expression of the elevated level of Hom-1.

8. The macrophage or monocyte for use of Claims 1 to 7, wherein said cancer is associated with tumor-associated macrophages (TAMs) that express a lower level of Hom-1 than macrophages in a control or a normal tissue of the subject having the cancer, optionally, said cancer is carcinoma, sarcoma, leukemia, osteosarcoma, lymphoma, melanoma, glioma, glioblastoma, pheochromocytoma, hepatoma, ovarian cancer, skin cancer, testicular cancer, gastric cancer, pancreatic cancer, renal cancer, breast cancer, prostate cancer, colorectal cancer, cancer of head and neck, brain cancer, esophageal cancer, bladder cancer, adrenal cortical cancer, lung cancer, bronchus cancer, thyroid cancer, endometrial cancer, nasopharyngeal cancer, cervical cancer, liver cancer, metastatic cancer, and cancer of unknown primary site.

9. The macrophage or monocyte for use of Claims 1 to 8, which is administered to the subject having the cancer through infusion or injection, such as intravenous, intrathecal, intramuscular, intraluminal, intratracheal, intraperitoneal, intracranial, subcutaneous, or transdermal administration.

10. The macrophage or monocyte for use of Claims 1 to 9, which is used in a combination therapy with radiation, chemotherapy, antibody therapy, or small molecule drug therapy.

## Patentansprüche

1. Makrophage oder Monozyt für die Verwendung bei der Behandlung eines Subjekts mit einem Krebs, wobei die Makrophage oder der Monozyt *ex vivo* induziert wird, um ein erhöhtes Niveau von endogenem Hom-1 zu exprimieren, oder wobei die Makrophage oder der Monozyt genetisch modifiziert ist, um ein erhöhtes Niveau von Hom-1 zu exprimieren, um Antitumoraktivität aufzuweisen.

2. Makrophage oder Monozyt für die Verwendung nach Anspruch 1, wobei die Makrophage oder der Monozyt wie folgt erzeugt wird: durch Einführen eines exogenen Expressionskonstrukts in eine Makrophage oder einen Monozyten, die/der von dem Subjekt mit dem Krebs stammt, oder durch Einführen eines exogenen Expressionskonstrukts in eine heterologe Makrophage oder einen heterologen Monozyten, die/der optional HLA-abgestimmt ist.

3. Makrophage oder Monozyt für die Verwendung nach Anspruch 1 oder Anspruch 2, wobei die Makrophage oder der Monozyt durch eine exogene Nukleinsäuresequenz genetisch modifiziert ist, die ein Hom-1-Polypeptid oder ein Fragment davon kodiert, das die Hom-1-Homöobox-Domäne enthält, wobei die Nukleinsäuresequenz funktionsfähig mit entweder einem endogenen Hom-1-Promotor oder mit einem heterologen Promotor verknüpft ist, wobei der heterologe Promotor optional ein konstitutiver Promotor oder ein induzierbarer Promotor ist.

4. Makrophage oder Monozyt für die Verwendung nach den Ansprüchen 1 bis 3, wobei die Makrophage oder der Monozyt *ex vivo* optional durch Folgende induziert wird: LPS, Choleratoxin (CTX), ein Chemotherapeutikum, Bestrahlung, ein Zytokin (z. B. GM-CSF), Phorbol-12-myristat-13-acetat (PMA) und einen Antikörper oder eine RNAi gegen einen Inhibitor von Hom-1-Expression, um ein höheres Niveau von endogenem Hom-1 zu exprimieren.

5. Makrophage oder Monozyt für die Verwendung nach den Ansprüchen 1 bis 4, wobei die Makrophage oder der Monozyt einen M1-Phänotyp aufweist oder erhöhte Niveaus von M1-Genen exprimiert, wie zum Beispiel IL1β, IL6, IL12, IL23, TNFα, iNOs, CD40, CD80, CD86, CD68, TLR4, TLR2, IL-1R, MHCII, CCL15, CCL20, CXCL9, CXCL1 oder SOCS3.

6. Makrophage oder Monozyt für die Verwendung nach den Ansprüchen 1 bis 5, wobei bei dem Subjekt mit dem Krebs vor der Behandlung bestimmt wird, dass ein niedrigeres Niveau von Hom-1-Expression in einer tumorassoziierten Makrophage (TAM) im Vergleich zu einer Kontrolle exprimiert wird.

7. Makrophage oder Monozyt für die Verwendung nach den Ansprüchen 1 bis 6, die eine Pro-Tumor-Makrophage ist, wie zum Beispiel eine tumorassoziierte Makrophage (TAM) oder eine M2-Makrophage, vor der induzierten oder genetisch modifizierten Expression des erhöhten Niveaus von Hom-1.

8. Makrophage oder Monozyt für die Verwendung nach den Ansprüchen 1 bis 7, wobei der Krebs mit tumorassoziierten Makrophagen (TAMs) assoziiert ist, die ein niedrigeres Niveau von Hom-1 exprimieren als Makrophagen in einer Kontrolle oder einem normalen Gewebe des Subjekts, das den Krebs hat, optional wobei der Krebs Folgendes ist: Karzinom, Sarkom, Leukämie, Osteosarkom, Lymphom, Melanom, Gliom, Glioblastom, Phäochromozytom, Hepatom, Eierstockkrebs, Hautkrebs, Hodenkrebs, Magenkrebs, Bauchspeicheldrüsenkrebs, Nierenkrebs, Brustkrebs, Prostatakrebs, kolorektaler Krebs, Krebs von Kopf und Hals, Gehirnkrebs, Speiseröhrenkrebs, Blasenkrebs, Nebennierenrindenkrebs, Lungenkrebs, Bronchialkrebs, Schilddrüsenkrebs, Endometriumkrebs, Nasopharynxkrebs, Gebärmutterhalskrebs, Leberkrebs, metastasierender Krebs und Krebs von unbekannter Primärstelle.

9. Makrophage oder Monozyt für die Verwendung nach den Ansprüchen 1 bis 8, die/der an das Subjekt mit dem Krebs durch Infusion oder Injektion verabreicht wird, wie zum Beispiel intravenöse, intrathekale, intramuskuläre, intraluminale, intratracheale, intraperitoneale, intrakranielle, subkutane oder transdermale Verabreichung.

10. Makrophage oder Monozyt für die Verwendung nach den Ansprüchen 1 bis 9, die/der in einer Kombinationstherapie mit Bestrahlung, Chemotherapie, Antikörpertherapie oder Therapie mit kleinmolekularen Arzneimitteln verwendet wird.

## Revendications

1. Macrophage ou monocyte destiné à une utilisation dans le traitement d'un sujet souffrant d'un cancer, le macrophage ou le monocyte étant induit *ex vivo* afin d'exprimer un taux élevé de Hom-1 endogène, où le macrophage ou le monocyte étant génétiquement modifié afin d'exprimer un taux élevé de Hom-1, pour présenter une activité antitumorale.

2. Macrophage ou monocyte pour une utilisation selon la revendication 1, le macrophage ou le monocyte étant généré par l'introduction d'une construction d'expression exogène dans un macrophage ou un monocyte dérivé du sujet souffrant du cancer, ou par l'introduction d'une construction d'expression exogène dans un macrophage ou un monocyte hétérologue qui est éventuellement HLA compatible.

3. Macrophage ou monocyte pour une utilisation selon la revendication 1 ou selon la revendication 2, le macrophage ou le monocyte étant génétiquement modifié par une séquence d'acide nucléique exogène codant pour un polypeptide Hom-1 ou un fragment de celui-ci qui contient le domaine d'homéoboîte de Hom-1, où la séquence d'acide nucléique est liée de manière opérante à un promoteur de Hom-1 endogène ou bien à un promoteur hétérologue, où le promoteur hétérologue est éventuellement un promoteur constitutif ou un promoteur inductible.

4. Macrophage ou monocyte pour une utilisation selon les revendications 1 à 3, le macrophage ou le monocyte étant induit *ex vivo,* éventuellement par un LPS, une toxine du choléra (CTX), un agent chimiothérapeutique, un rayonnement, une cytokine (par exemple GM-CSF), le 12-myristate 13-acétate de phorbol (PMA), un anticorps ou un ARNi contre un inhibiteur de l'expression de Hom-1, afin d'exprimer un taux supérieur de Hom-1 endogène.

5. Macrophage ou monocyte pour une utilisation selon les revendications 1 à 4, le macrophage ou le monocyte présentant un phénotype M1 ou exprimant des taux élevés de gènes M1 tels qu'IL-1β, IL6, IL12, IL23, TNFα, iNOs, CD40, CD80, CD86, CD68, TLR4, TLR2, IL-1R, MHCII, CCL15, CCL20, CXCL9, CXCL1, ou SOCS3.

6. Macrophage ou monocyte pour une utilisation selon les revendications 1 à 5, le sujet souffrant du cancer, préalablement audit traitement, ayant été déterminé comme exprimant un taux inférieur d'expression de Hom-1 dans un macrophage associé aux tumeurs (TAM) par rapport à un témoin.

7. Macrophage ou monocyte pour une utilisation selon les revendications 1 à 6, qui est un macrophage pro-tumoral, tel qu'un macrophage associé aux tumeurs (TAM) ou un macrophage M2, préalablement à l'expression induite ou génétiquement modifiée du taux élevé de Hom-1.

8. Macrophage ou monocyte pour une utilisation selon les revendications 1 à 7, ledit cancer étant associé avec les macrophages associés aux tumeurs (TAM) qui expriment un taux inférieur de Hom-1 par rapport aux macrophages dans un tissu témoin ou normal du sujet souffrant du cancer, éventuellement, ledit cancer étant un carcinome, un sarcome, une leucémie, un ostéosarcome, un lymphome, un mélanome, un gliome, un glioblastome, un phéochromocytome, un hépatome, un cancer de l'ovaire, un cancer de la peau, un cancer du testicule, un cancer de l'estomac, un cancer du pancréas, un cancer du rein, un cancer du sein, un cancer de la prostate, un cancer colorectal, un cancer de la tête et du cou, un cancer du cerveau, un cancer de l'œsophage, un cancer de la vessie, un cancer corticosurrénalien, un cancer du poumon, un cancer des bronches, un cancer de la thyroïde, un cancer de l'endomètre, un cancer nasopharyngien, un cancer du col de l'utérus, un cancer du foie, un cancer métastatique, et un cancer d'origine primitive inconnue.

9. Macrophage ou monocyte pour une utilisation selon les revendications 1 à 8, qui est administré au sujet souffrant du cancer par perfusion ou injection, tel que par administration intraveineuse, intrathécale, intramusculaire, intraluminale, intratrachéale, intrapéritonéale, intracrânienne, sous-cutanée, ou transdermique.

10. Macrophage ou monocyte pour une utilisation selon les revendications 1 à 9, qui est utilisé dans une thérapie associative avec un rayonnement, une chimiothérapie, une thérapie par anticorps, ou une thérapie par médicaments à petites molécules.


### SEQUENCE LISTING

<110> Zu, Zhenglun
<120> METHOD OF MODIFYING MACROPHAGE DIFFERENTIATION AND IMMUNITY
<130> 218008-0005PCT
<150> US 62/253,836
   <151> 2015-11-11
<160> 2
<170> PatentIn version 3.5
<210> 1
   <211> 2428
   <212> DNA
   <213> Homo sapiens
<220>
   <221> CDS
   <222> (12)..(788)
   <223> Hom-1
<400> 1
   acctggccgc c atg cgc ctc tcc tcc tcc cca cct cgt ggc ccg cag cag     50
                Met Arg Leu Ser Ser Ser Pro Pro Arg Gly Pro Gln Gln
                1               5                   10
   ctc tcc agc ttt ggc tcc gtg gac tgg ctc tcc cag agc agc tgc tca          98
   Leu Ser Ser Phe Gly Ser Val Asp Trp Leu Ser Gln Ser Ser Cys Ser
       15                  20                  25
   ggg ccg acc cac acc ccc agg cct gcc gac ttc tcc ctg ggg agc ctc         146
   Gly Pro Thr His Thr Pro Arg Pro Ala Asp Phe Ser Leu Gly Ser Leu
   30                  35                  40                  45
   cct ggc cca ggc cag aca tcc ggc gcc cgg gag ccc cct cag gcc gtc         194
   Pro Gly Pro Gly Gln Thr Ser Gly Ala Arg Glu Pro Pro Gln Ala Val
                   50                  55                  60
   agc atc aag gag gcc gcc ggg tcc tca aat ctg cct gcg ccg gag agg         242
   Ser Ile Lys Glu Ala Ala Gly Ser Ser Asn Leu Pro Ala Pro Glu Arg
               65                  70                  75
   acc atg gcc ggg ttg agt aag gag cca aat acc ttg cgg gcc ccc cgt         290
   Thr Met Ala Gly Leu Ser Lys Glu Pro Asn Thr Leu Arg Ala Pro Arg
           80                  85                  90
   gtc cgc aca gcc ttc acc atg gag cag gtc cgc acc ttg gag ggc gtc         338
   Val Arg Thr Ala Phe Thr Met Glu Gln Val Arg Thr Leu Glu Gly Val
       95                  100                 105
   ttc cag cac cac cag tac ctg agc cct ctg gag cgg aag agg ctg gcc         386
   Phe Gln His His Gln Tyr Leu Ser Pro Leu Glu Arg Lys Arg Leu Ala
   110                 115                 120                 125
   agg gag atg cag ctc tca gag gtc cag ata aaa acc tgg ttt cag aat         434
   Arg Glu Met Gln Leu Ser Glu Val Gln Ile Lys Thr Trp Phe Gln Asn
                   130                 135                 140
   cgc cgc atg aaa cac aaa cgg caa atg cag gac ccc cag ctg cac agc        482
   Arg Arg Met Lys His Lys Arg Gln Met Gln Asp Pro Gln Leu His Ser
               145                 150                 155
   ccc ttc tcg ggg tct ctc cat gcg ccc cca gct ttc tac tca acg tct        530
   Pro Phe Ser Gly Ser Leu His Ala Pro Pro Ala Phe Tyr Ser Thr Ser
           160                 165                 170
   tct ggc ctt gcc aat ggc ctg cag ctg ctg tgc cct tgg gca ccc ctg        578
   Ser Gly Leu Ala Asn Gly Leu Gln Leu Leu Cys Pro Trp Ala Pro Leu
       175                 180                 185
   tcc ggg ccc cag gct ctg atg ctg ccc cct ggc tcc ttc tgg ggt ctc        626
   Ser Gly Pro Gln Ala Leu Met Leu Pro Pro Gly Ser Phe Trp Gly Leu
   190                 195                 200                 205
   tgc caa gtg gca caa gag gcc ctg gca tct gcg gga gct tcc tgc tgc        674
   Cys Gln Val Ala Gln Glu Ala Leu Ala Ser Ala Gly Ala Ser Cys Cys
                   210                 215                 220
   ggg cag cct ctg gcg tcc cac ccc cct acc cca ggc cgg cct tcg ctg        722
   Gly Gln Pro Leu Ala Ser His Pro Pro Thr Pro Gly Arg Pro Ser Leu
               225                 230                 235
   gga cca gcc ctg tcc acg ggg ccc cgg ggc ctg tgt gct atg cca cag        770
   Gly Pro Ala Leu Ser Thr Gly Pro Arg Gly Leu Cys Ala Met Pro Gln
           240                 245                 250
   acg ggg gat gca ttt tga ggaggcacct ctgactccca cactcgcggt               818
   Thr Gly Asp Ala Phe
       255
   cttgctgatc gcacctggct cctacctgga ggactcagtt gttctgttta catcctggtg      878
   gcacctctca ccctgaccca cacaaaggtt ctggagatta ctggagaata tatataaata      938
   tatatatgta cgtatatatg taaatacaca tatacgtata tataaatata tatatacata      998
   tgtgtgtgta tatatatata tatttttttt tttttttttt tttttgagac ggagtgttgc     1058
   tctgtcaccc aggctggagt gcaatgacgc aatctcggct cactgcaacc tccgcctcct     1118
   gggttcaagc gattctccag cctcagcctc ccgagtagct gggattacag acacccgcca     1178
   ccacgcccgg ctaatttttt ctatttttag tagaaatggg gtttcaccat gttagccagg     1238
   ctggtctcaa actcctgacc ctgtgatccg cccgcctcgg cctcccaaag tgctgggatt     1298
   acaggcatga gccactgcac ccggccctga gaatatattt attaaagcca cctcttcact     1358
   gaaagttacc gaaagagtcg gtttaggaag gaaacgaagg gtcagtgaac agagtcaaat     1418
   gcagaagtgg gcttgtcatg ggtagggctt tcggcgtacg ataaaaggat catttgtttt     1478
   ttaaaagggg ttggaaaaac tggttttcca gttggaaaca gtaaaggttg taagctttgt     1538
   gtgtacaaaa gaaaacaggg aatgcaggtg tgtttatagc gttgtggttc aagtccctct     1598
   taacaagaac tccaaagctg gaaagcagga gggaacaaag gtgaacatga aggcgaggat     1658
   gctggggccc tgcagtgcgc tctaggctgt gcgtgagccg ggactgtacc cacagcttgc     1718
   tgagggctgc tcttcttggg ccagggaaag cagggcagcc gggacctgcg gctgtgcctg     1778
   gactgaagct gtcccgcagg tccccaccct ccaacacgtg ctcacctgtc cccctcctcg     1838
   cagcagcctc gggacaaaac aatgactcaa ggacagcact tctcgcagaa ggtctggaag     1898
   tgcccagaat gggaggcacg gaagcccctc ccggggagga ctcccgcgtt gatggaccgt     1958
   tcttggtgca gactcctgac tgcgtgcatg aaacctgaga caagtgcaat tccttccatg     2018
   tcgccccaga gtgcccagga ggcaggcagt gcggggtgcc caggcagacg ggttcagcct     2078
   gcagaactgg aggcgacctg tgaaacccac ccgggcaccc caacaggaac agaagcgtgg     2138
   tcctgcggct gcgtccccag cgagtttcac tttccccttg ctcgtttctc ccttgttgta     2198
   agtgtttaca actggcatgt gcttttaaac gtcaggtaag aggggaacag ctgctgtaca     2258
   tcgtcctggc gagtgacaat gtgacagaag cctgggcgag gccctcggag ggcagcagct     2318
   ggacaggggc tactgggttt ggcctggaca gcactgattt gtggatgtgg atgggggcac     2378
   gttgtccgtg ataaaagtac aagtgcccct cacaaaaaaa aaaaaaaaaa                2428
<210> 2
   <211> 258
   <212> PRT
   <213> Homo sapiens
<400> 2
   Met Arg Leu Ser Ser Ser Pro Pro Arg Gly Pro Gln Gln Leu Ser Ser
   1               5                   10                  15
   Phe Gly Ser Val Asp Trp Leu Ser Gln Ser Ser Cys Ser Gly Pro Thr
               20                  25                  30
   His Thr Pro Arg Pro Ala Asp Phe Ser Leu Gly Ser Leu Pro Gly Pro
           35                  40                  45
   Gly Gln Thr Ser Gly Ala Arg Glu Pro Pro Gln Ala Val Ser Ile Lys
       50                  55                  60
   Glu Ala Ala Gly Ser Ser Asn Leu Pro Ala Pro Glu Arg Thr Met Ala
   65                  70                  75                  80
   Gly Leu Ser Lys Glu Pro Asn Thr Leu Arg Ala Pro Arg Val Arg Thr
                   85                  90                  95
   Ala Phe Thr Met Glu Gln Val Arg Thr Leu Glu Gly Val Phe Gln His
               100                 105                 110
   His Gln Tyr Leu Ser Pro Leu Glu Arg Lys Arg Leu Ala Arg Glu Met
           115                 120                 125
   Gln Leu Ser Glu Val Gln Ile Lys Thr Trp Phe Gln Asn Arg Arg Met
       130                 135                 140
   Lys His Lys Arg Gln Met Gln Asp Pro Gln Leu His Ser Pro Phe Ser
   145                 150                 155                 160
   Gly Ser Leu His Ala Pro Pro Ala Phe Tyr Ser Thr Ser Ser Gly Leu
                   165                 170                 175
   Ala Asn Gly Leu Gln Leu Leu Cys Pro Trp Ala Pro Leu Ser Gly Pro
               180                 185                 190
   Gln Ala Leu Met Leu Pro Pro Gly Ser Phe Trp Gly Leu Cys Gln Val
           195                 200                 205
   Ala Gln Glu Ala Leu Ala Ser Ala Gly Ala Ser Cys Cys Gly Gln Pro
       210                 215                 220
   Leu Ala Ser His Pro Pro Thr Pro Gly Arg Pro Ser Leu Gly Pro Ala
   225                 230                 235                 240
   Leu Ser Thr Gly Pro Arg Gly Leu Cys Ala Met Pro Gln Thr Gly Asp
                   245                 250                 255
   Ala Phe